# EUROPEAN PATENT SPECIFICATION

(11) **EP 2 381 853 B1**
(45) Date of publication and mention of the grant of the patent: **13.01.2016**
(21) Application number: 09755912.4
(22) Date of filing: 20.11.2009
(51) Int. Cl.: A61B 17/02

(54) **SURGICAL INSTRUMENT FOR OPERATIONS ON THE SPINAL COLUMN**
OPERATIONSINSTRUMENT FÜR OPERATIONEN AN DER WIRBELSÄULE
INSTRUMENT CHIRURGICAL POUR DES INTERVENTIONS SUR LA COLONNE VERTÉBRALE

(30) Priority: 21.11.2008 IT MI20082078
(43) Date of publication of application: 02.11.2011
(73) Proprietor: Calvosa, Giuseppe, 56123 Pisa (IT); N.B.R. New Biotechnology Research, 37121 Verona (IT)
(72) Inventor: TENUCCI, Miria, I-55100 Lucca (IT); CASELLA, Renato, I-56019 Vecchiano (IT); CALVOSA, Giuseppe, II-56123 Pisa (IT)
(74) Representative: Bergamini, Silvio
(86) International application number: PCT/EP2009/065558
(87) International publication number: WO 2010/057979

(56) References cited:
- WO-A-00/44320
- WO-A-2005/077288
- WO-A-2008/153732
- JP-A- 2005 296 562
- US-A- 4 686 972
- US-B1- 6 500 206
- US-B1- 7 442 195

## Description

### Technical field

The present invention relates to a surgical instrument for operations on the spinal column. More particularly, the invention relates to a surgical instrument that allows to expose portions of spinal column on which for example prostheses or screws are to be implanted subsequently.

### Background Art

As is known, operations on the spinal column, particularly for implanting prostheses, screws and the like, require parting the muscles from the spinal column by using a periosteal elevator, which allows to cut the muscles closely in contact with the spinal column so as to be able to then part it completely from the column.

In this manner, the muscle bundles separate from the spinal column, allowing the surgeon to have free access to the spinal column in order to implant fixation screws, prostheses and the like.

Currently, the technique for parting muscles from the spinal column therefore entails using a scalpel to cut the muscles at the region of contact of the muscles with the spinal column and the periosteal elevator to separate the muscles from the bone. This procedure is highly invasive, since parting of the muscles by resecting and parting it entails considerable bleeding. Document US 6500206 discloses an instrument for inserting spinal vertebral implant.

### Disclosure of the Invention

The aim of the present invention is to provide a surgical instrument for operations on the spinal column that allows the surgeon to part easily the muscles from the spinal column while minimizing bleeding.

Within this aim, an object of the present invention is to provide a surgical instrument for operations on the spinal column that allows the surgeon, with a combined action of his hands, to avoid the use of the periosteal elevator except in the initial step for parting the muscles from the spinal column.

Another object of the present invention is to provide a surgical instrument that is highly reliable, relatively simple to provide and at competitive costs.

This aim, these objects and others which will become better apparent hereinafter are achieved by a surgical instrument for operations on the spinal column, characterized in that it comprises a handle body which ends, at one end, with a substantially flat portion which blends with the body of the instrument, said substantially flat portion being adapted to push on a gauze for tamponing a wound.

### Brief description of the drawings

Further characteristics and advantages of the invention will become better apparent from the following detailed description of some preferred but not exclusive embodiments of the surgical instrument according to the present invention, illustrated by way of non-limiting example in the accompanying drawings, wherein:
Figure 1 is a perspective view of a first embodiment of the surgical instrument according to the present invention;
Figure 2 is a side view of the surgical instrument according to the present invention;
Figure 3 is a perspective view of a second embodiment of the surgical instrument;
Figure 4 is a perspective view of a gauze adapted to be used with the surgical instrument according to the present invention;
Figure 5 illustrates the operating sequence of use of the surgical kit according to the present Invention.

### Ways of carrying out the Invention (CLEAR COPY)

With reference to the figures, a surgical Instrument according to the present invention, generally designated by the reference numeral 1, comprises a handle 2 constituted by a plate-like body that has a substantially curved or rectilinear shape which ends at one end with a portion that has two curvilinear parts 3a, 3b which join with the body portion, laterally thereto, and end substantially with a flat portion 4 that joins the two curvilinear parts 3a, 3b.

In a side view, the end portion, generally designated by the reference numeral 5, has a substantially wedge-like shape and tapers therefore toward the portion that lies furthest from the handle 2.

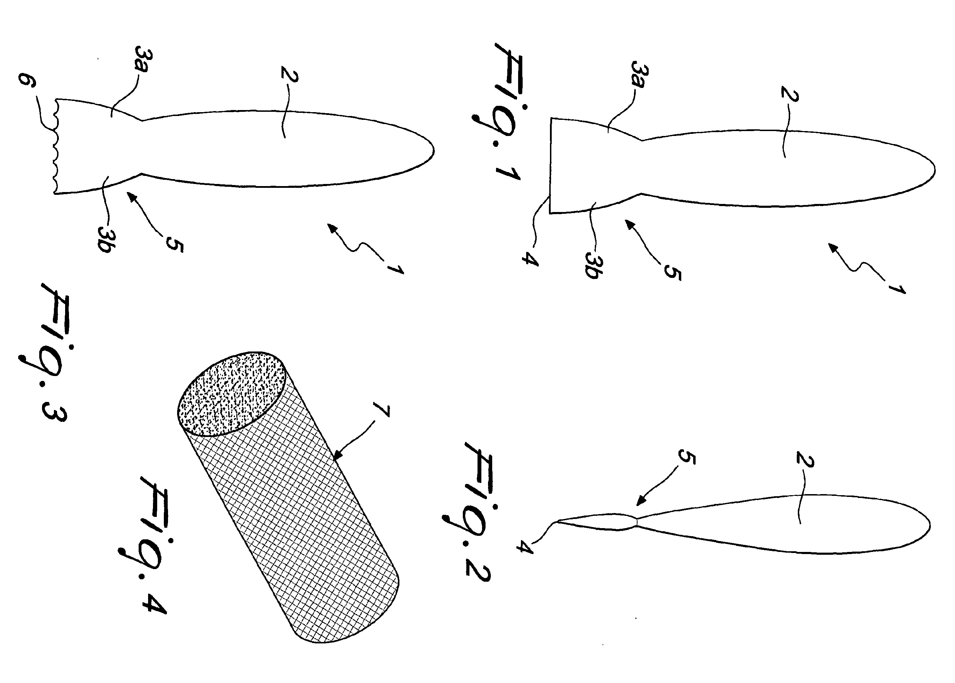

Conveniently, the rectilinear portion 4 can have a set of teeth 6, as shown in Figure 3.

Conveniently, the surgical instrument according to the invention is adapted to be used with elongated gauzes 7 of the cylindrical type, as shown in Figure 4, which have different sizes and are in any case adapted to roll on the bone, parting and separating the overlying muscles.

Advantageously, the surgical instrument 1 allows a surgeon to insert the elongated gauze 7, or any cylindrical support on which an elongated gauze is rolled, pushing it within the slit created by the scalpel and/or periosteal elevator during the resection of the muscles "M" of the spinal column "SC", and to then push the gauze 7 downward, advantageously parting the muscles "M" from the remaining part of the spinal column "SC", acting directly with the surgical instrument 1, without the aid of the scalpel and/or periosteal elevator.

, Substantially, the surgeon, by acting with the surgical instrument 1, pushes the elongated gauze 7 downward, adjacent to the spinal column "SC", between the column and the muscles "M" to be parted, thus parting the remaining portion of muscles that had not been parted beforehand by the action of the scalpel.

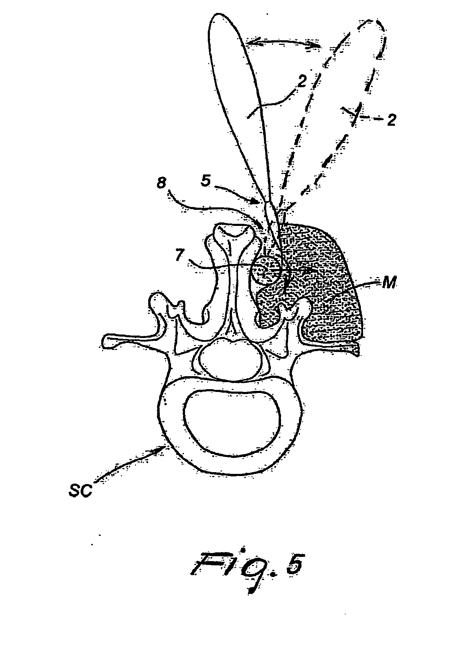

This operation can be performed more easily by using two surgical instruments 1 according to the invention simultaneously, one held in each hand, and by performing a combined and alternating action of the two instruments, so as to push the elongated gauze 7 within the slit created by the scalpel (Figure 5).

The possibility to insert the elongated gauze 7 directly in the slit created by the action of the scalpel by means of the surgical instrument according to the invention allows to part the muscles "M" from the bone and absorb most of the blood that exits due to the parting of the muscles from the spinal column "SC", at the same time performing hemostasis by direct compression and by indirect action, thanks to the substances with which the elongated gauze 7 can be impregnated, i.e., platelet gel, fibrin glue or other hemostatic substance.

In this manner, this step of the surgical operation is far easier to perform for the surgeon and substantially less invasive for the patient.

In practice it has been found that the surgical instrumental kit according to the present invention fully achieves the intended aim and objects, since it allows to part the muscles "M" from the spinal column "SC" by using the scalpel only initially, subsequently resorting to a mechanical action of pushing the gauze into the slit created by the scalpel.

The surgical instrumental kit thus conceived is susceptible of numerous modifications and variations, all of which are within the scope of the appended claims; all the details may further be replaced with other technically equivalent elements.

In practice, the materials used, as well as the contingent shapes and dimensions, may be any according to requirements and to the state of the art.

Where technical features mentioned in any claim are followed by reference signs, those reference signs have been included for the sole purpose of increasing the intelligibility of the claims and accordingly, such reference signs do not have any limiting effect on the interpretation of each element identified by way of example by such reference signs.

## Claims

1. A surgical instrumental kit for an operations on the human spinal column comprising:
- An elongated gauze (7), and
- A surgical instrument (1), said surgical instrument (1) comprising:
- A handle body (2), and
- A substantially flat portion (4) joined with said handle body (2) of the surgical instrument (1),
**characterized in that** said flat portion (4) has a wedge-like shaped end portion (5) defining a rectilinear portion, said rectilinear portion being designed to urge said elongated gauze (7) to roll on a bone, to part and to separate overlying muscles.

2. A surgical instrumental kit according to claim 1, wherein said substantially flat portion (4) comprises a pair of curved portions (3a, 3b) shaped to blend with the body (2).

3. A surgical instrumental kit according to claim 1, wherein said handle body (2) is a plate-like element with a substantially curved configuration.

4. A surgical instrumental kit according to one or more of preceding claims, wherein said substantially flat portion (4) has a plurality of teeth (6).

5. A surgical instrumental kit according to one or more of preceding claims, wherein said substantially flat portion (4) is substantially wedge-shaped in transverse cross section.

6. A surgical instrumental kit according to claim 1, wherein said elongated gauze is a cylindrical roll (7) of gauze.

## Patentansprüche

1. Chirurgisches Instrumenten-Besteck für eine Operation an der menschlichen Wirbelsäule mit
- einem verlängerten Verbandmull (7) und
- einem chirurgischen Instrument (1), wobei dieses chirurgische Instrument (1) umfasst:
- ein Griffgehäuse (2) und
- einen im Wesentlichen flachen Abschnitt (4), der verbunden ist mit dem Griffgehäuse (2) des chirurgischen Instruments (1),
**dadurch gekennzeichnet, dass** der flache Abschnitt (4) einen keilartig geformten Endabschnitt (5) aufweist, der einen geradlinigen Abschnitt bestimmt, wobei dieser geradlinige Abschnitt ausgelegt ist, den verlängerten Verbandmull (7) dazu zu drängen, auf einen Knochen zu rollen, um aufliegende Muskeln zu lösen und zu trennen.

2. Chirurgisches Instrumenten-Besteck gemäß Anspruch 1, **dadurch gekennzeichnet, dass** der flache Abschnitt (4) ein Paar gekrümmter Abschnitte (3a, 3b) umfasst, die geformt sind in das Gehäuse (2) zu verlaufen.

3. Chirurgisches Instrumenten-Besteck gemäß Anspruch 1, **dadurch gekennzeichnet, dass** das Griffgehäuse (2) ein plattenartiges Element mit einer im Wesentlichen gekrümmten Auslegung ist.

4. Chirurgisches Instrumenten-Besteck gemäß einem oder mehreren der vorhergehenden Ansprüche, **dadurch gekennzeichnet, dass** der im Wesentlichen flache Abschnitt (4) eine Vielzahl von Zähnen (6) aufweist.

5. Chirurgisches Instrumenten-Besteck gemäß einem oder mehreren der vorhergehenden Ansprüche, **dadurch gekennzeichnet, dass** der im Wesentlichen flache Abschnitt (4) im Wesentlichen keilförmig ist im transversalen Querschnitt.

6. Chirurgisches Instrumenten-Besteck gemäß Anspruch 1, **dadurch gekennzeichnet, dass** der verlängerte Verbandmull (7) eine zylindrische Rolle aus Gewebe ist.

## Revendications

1. Jeu d'instruments chirurgicaux pour une intervention sur la colonne vertébrale humaine, comprenant :
- une gaze allongée (7), et
- un instrument chirurgical (1), ledit instrument chirurgical (1) comprenant :
- un corps de poignée (2), et
- une partie sensiblement plate (4) unie audit corps de poignée (2) de l'instrument chirurgical (1),
**caractérisé en ce que** ladite partie sensiblement plate (4) comporte une partie terminale cunéiforme (5) définissant une partie rectiligne, ladite partie rectiligne étant conçue pour faire rouler ladite gaze allongée (7) sur un os, pour écarter et séparer les muscles sus-jacents.

2. Jeu d'instruments chirurgicaux selon la revendication 1, dans lequel ladite partie sensiblement plate (4) comprend une paire de parties courbées (3a, 3b) façonnées de manière à s'intégrer au corps (2).

3. Jeu d'instruments chirurgicaux selon la revendication 1, dans lequel ledit corps de poignée (2) est un élément en forme de plaque ayant une configuration sensiblement courbée.

4. Jeu d'instruments chirurgicaux selon une ou plusieurs des revendications précédentes, dans lequel ladite partie sensiblement plate (4) comporte une pluralité de dents (6).

5. Jeu d'instruments chirurgicaux selon une ou plusieurs des revendications précédentes, dans lequel ladite partie sensiblement plate (4) est sensiblement cunéiforme en coupe transversale.

6. Jeu d'instruments chirurgicaux selon la revendication 1, dans lequel ladite gaze allongée est un rouleau cylindrique (7) de gaze.
